# EUROPEAN PATENT SPECIFICATION

(11) **EP 2 307 575 B1**
(45) Date of publication and mention of the grant of the patent: **11.10.2017**
(21) Application number: 09794720.4
(22) Date of filing: 04.06.2009
(51) Int. Cl.: C12Q 1/68, A61K 48/00, C12N 15/79, C12P 19/34, C12N 15/64

(54) **UNPROCESSED ROLLING CIRCLE AMPLIFICATION PRODUCT**
UNVERARBEITETES ROLLING-CIRCLE-AMPLIFIKATIONSPRODUKT
PRODUIT D'AMPLIFICATION PAR CERCLE ROULANT NON TRAITÉ

(30) Priority: 09.07.2008 US 169993
(43) Date of publication of application: 13.04.2011
(73) Proprietor: General Electric Company, Schenectady, NY 12345 (US)
(72) Inventor: DAVIS, Brian, M., Niskayuna, New York 12309-1027 (US); NELSON, John, R., Niskayuna, New York 12309-1027 (US); TORRES, Andrew, S., Niskayuna, New York 12309-1027 (US); WOOD, Nichole, L., Niskayuna, New York 12309-1027 (US)
(74) Representative: Aldenbäck, Ulla Christina
(86) International application number: PCT/SE2009/050667
(87) International publication number: WO 2010/005365

(56) References cited:
- WO-A2-2004/096288
- WO-A2-2006/063355
- WO-A2-2007/018744
- CN-A- 101 103 122
- US-A1- 2002 187 952
- MIZUTA R. ET AL: 'Atomic force microscopy analysis of rolling circle amplification of plasmid DNA' ARCH. HISTOL. CYTOL. vol. 66, no. 2, 2003, pages 175 - 181, XP003026034
- DEMIDOV V.: '10 years of rolling the minicircles: RCA assays in DNA diagnostics' EXPERT REV. MOL. DIAGN. vol. 5, no. 4, 2005, pages 477 - 478, XP009074526
- KENDIRGI F. ET AL: 'Novel linear DNA vaccines induce protective immune responses against lethal infection with influenza virus type A/H5N1' HUM. VACCIN vol. 4, no. 6, 2008, pages 410 - 419, XP003026035

## Description

### TECHNICAL FIELD

The invention relates generally to DNA replication and to the use of an unprocessed (non-cleaved, non-circularized, and non-supercoiled) rolling circle amplification (RCA) product. Such use comprises, for example, use in the production of a DNA vaccine, in the elicitation of an immune response in an organism, and in the transfection of a living cell.

### BACKGROUND OF THE INVENTION

Rolling circle amplification (RCA) has been employed in the replication of circularized DNA sequences for some time. RCA products have been used in DNA sequencing, cloning, library construction, probe generation, and genetic screening. More recently, it has been proposed that RCA products be employed in cellular expression, wherein cells are administered naked DNA to produce an RNA or protein, and in DNA vaccination, wherein an organism is administered naked DNA to produce an immunological response. In the case of DNA vaccination, rather than administering the pathogen itself (generally in a dead or disabled form so as to minimize any risk of actual infection), as in standard vaccinations, some portion of the pathogen's genome is administered and is then replicated and expressed by the organism in a manner sufficient to elicit an immune response. Because only a portion of the pathogen's genome is administered, there is little or no risk of actual pathogenic infection.

To date, DNA to be used for cell transfection or DNA vaccines has generally been made using plasmid DNA. However, the use of plasmid DNA requires labor-intensive and expensive plasmid purification and runs the risk of contamination by extraneous bacterial components such as proteins, DNA, RNA, small molecules, or purification reagents (e.g., ethidium bromide, chloroform, phenol, etc.). Any of these contaminants can have undesirable consequences. WO2007/018744 A2 describes a method for producing processed RCA products which can be used in DNA based therapeutics. Mizuta et al., Arch. Histol. Cytol., 66(2) pp. 175-181, 2003, describes the use of unprocessed RCA amplified plasmid DNA from bacteria for transfection into COS7 cells.

Methods developed more recently employ cell-free RCA techniques to avoid the need for such plasmid purification and risk of contamination, and are therefore better suited for expression in cellular systems or therapeutic applications, such as in DNA vaccines. However, in order to ensure sufficient uptake and expression in the DNA recipient, such techniques have, to date, required extensive post-amplification processing of the RCA product, wherein the product is broken into shorter units (monomers, dimers, trimers, etc.) and then circularized or supercoiled. Such processing adds considerable time and expense to the production of an RCA product suitable for use in expression or therapeutic applications.

### BRIEF DESCRIPTION OF THE INVENTION

There is a need to overcome deficiencies in the art, including those described above. In particular, there is a need for physiologically-effective RCA products that do not require post-amplification processing, as well as methods for their production and use.

Embodiments of the disclosure provide methods related to, and compositions comprising, unprocessed (i.e., not deliberately or intentionally cleaved, circularized, and/or supercoiled) rolling circle amplification (RCA) product. The term "unprocessed," as used herein, does not include heat denaturation to irreversibly inactivate enzymatic activity, or purification methods to change DNA concentration, move DNA into a different solution, or remove RCA reaction components from the RCA product.

A first aspect of the invention provides a method of producing a rolling circle amplification (RCA) product as defined in the appendend claims.

A second aspect of the invention provides a DNA vaccine comprising at least one unprocessed rolling circle amplification (RCA) product suitable for administration to an organism.

### BRIEF DESCRIPTION OF THE DRAWINGS

These and other features of embodiments of the invention will be more readily understood from the following detailed description of the various aspects of the invention taken in conjunction with the accompanying drawings that depict various embodiments of the invention, in which:
FIG. 1 shows a schematic flow diagram of select stages of an illustrative method of producing an RCA product suitable for use according to aspects of the invention.
FIGS. 2-4 show graphs of the transfection efficiencies of unprocessed RCA product in comparison with plasmid RCA product.
FIG. 5 shows a flow diagram of an illustrative method of preparing and using unprocessed RCA product to transfect a living cell according to an embodiment of the invention.

It is noted that the drawings are not to scale. The drawings are intended to depict only typical aspects of the invention, and therefore should not be considered as limiting the scope of the invention. In the drawings, like numbering represents like elements between the drawings.

### DETAILED DESCRIPTION OF THE INVENTION

As noted above, the subject matter disclosed herein relates generally to DNA replication and, more particularly, to the production and use of an unprocessed (non-cleaved, non-circularized, and non-supercoiled) rolling circle amplification (RCA) product. Some embodiments of the invention relate to the use of an unprocessed rolling circle amplification (RCA) product. In one aspect of the invention, such use includes use of the unprocessed RCA product in a DNA vaccine. In other aspects, such use includes use in eliciting an immune response in an organism and/or in transfecting the unprocessed RCA product into a living cell. These RCA-transfected living cells may be used for protein expression, cell therapy, or other research or medical uses. An illustrative use in protein expression includes the expression of some signal (e.g., expression of a green fluorescent protein) useful in tracking a cell (e.g., during cell therapy). These examples are not meant to limit the application of such uses and are merely illustrative. The unprocessed RCA products when used in living cells may be adapted for many different applications, all of which are within the scope of the invention.

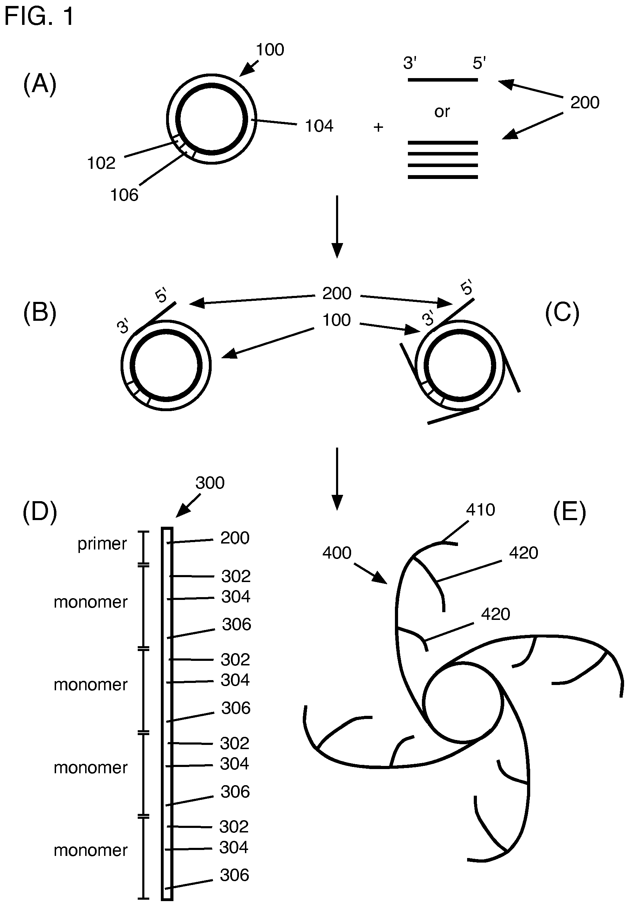

FIG. 1 shows a schematic flow diagram of select stages of an illustrative method of producing an RCA product suitable for use according to aspects of the invention. In (A), a circular DNA template 100 (which may be single-stranded or double-stranded) including a promoter sequence 102, a target sequence 104, and a termination sequence 106, is combined with one or a plurality of oligonucleotide primers 200. (While described here as employing a DNA template, it should be noted that suitable methods may similarly employ an RNA template.) Target sequence 104 may be any DNA sequence, the replication of which is desired. Typically, target sequence 104 is derived from a bacterium, a virus, a fungus, a parasitic organism, or a non-parasitic organism and will code for an expression product (e.g., a protein, such as a surface antigen) capable of eliciting an immune response in an organism, if that organism were to be exposed to the bacterium, virus, fungus, parasitic, or non-parasitic organism. In some embodiments, target sequence 104 codes for a protein, a messenger RNA (mRNA) sequence, a non-coding RNA sequence, a micro RNA (miRNA) sequence, a small interfering RNA (siRNA) sequence, or a monoclonal antibody (mAb) chain (heavy or light chain).

In some embodiments of the invention, the circular DNA template 100 may include multiple promoter, target, and termination sequences. In such a case, target sequences for different strains of the same bacterium, virus, etc. may be transferred to a host cell or organism. Alternatively, entirely unrelated target sequences may be transferred in the same unprocessed RCA product. Such embodiments may be particularly valuable where the unprocessed RCA product is to be used in a DNA vaccine, enabling vaccination against multiple strains and/or diseases in a single vaccine.

In some embodiments of the invention, the circular DNA template is an expression plasmid containing a sequence needed for replication and selection in host bacterial cells, in addition to the target sequence. This sequence can be useful in terms of enabling the initial creation of the recombinant DNA circular construct that can subsequently be used as a template for prepared or bulk amounts of DNA by RCA.

At least a portion of the sequence(s) of oligonucleotide primers 200 is complementary to a portion of the sequence of circular DNA template 100. In some embodiments, the complementary portion of the oligonucleotide primer sequence is located at the 3' end of the primer while a non-complementary portion of the sequence is located at the 5' end. Such a primer may aid in displacement of the primer during RCA, as will be described in greater detail below. It is understood that RCA reactions require a primer, and that RCA reactions can be initiated by either added oligonucleotide primer(s) or primer(s) synthesized in the RCA reaction. Such synthesized primers can be generated by the use of primase in the RCA reaction.

In (B), a single oligonucleotide primer 200 is shown annealed to the circular DNA template 100. Such annealing is typical where a single "species" of primer is used, each primer having the same sequence, which is complementary to only a single portion of the circular DNA template 100. Alternatively, as shown in (C), a plurality of "species" of primers may be employed, the plurality including sequences complementary to more than one portion of the circular DNA template 100. As shown in (C), four primers 200 are used, each having a sequence complementary to a different portion of the circular DNA template 100. In the case that multiple "species" of primers are used, such primers may be specifically designed to include sequences complementary to a portion of the circular DNA template 100 or may include random DNA sequences. Multi-primed RCA is described in US Patent No. 6,323,009 to Lasken et al. Commercially-available amplification kits, such as the TempliPhi™ Amplification Kits available from GE Healthcare, may also be employed in practicing aspects of the invention.

In either case, replication of the circular DNA template 100 continues, with the DNA polymerase displacing the newly-replicated, single-stranded DNA from the circular DNA template 100. The result is a linear concatemer of the sequence of the circular DNA template 100. Primers bind to the displaced single-stranded DNA and complete the double-stranded RCA product.

Any number of polymerases may be employed in RCA. Among the more commonly employed DNA polymerases are those exhibiting 3'-5' exonuclease activity, such as, for example, bacteriophage Phi29 DNA polymerase, Tts DNA polymerase, phage M2 DNA polymerase, bacterial DNA polymerase I (Pol I), the Klenow fragment of DNA polymerase I, bacterial DNA polymerase III (Pol III), T5 DNA polymerase, PRD1 DNA polymerase, T4 DNA polymerase holoenzyme, T7 DNA polymerase, and Bst DNA polymerase I. Among these, bacteriophage Phi29 DNA polymerase is particularly preferred in some embodiments of the invention. Polymerases not exhibiting 3'-5' exonuclease activity may also be used, including, for example, Taq polymerase, Tfl polymerase, Tth polymerase, eukaryotic DNA polymerase alpha, and DNA polymerases modified to eliminate 3'-5' exonuclease activity.

In (D), a double-stranded linear RCA product 300 is shown, as may be obtained by the annealing of a single "species" of primer, as in (B). Linear RCA product 300 includes the primer sequence 200 and a plurality of monomers, linear copies of the sequence of circular DNA template 100 (i.e., repeating units of promoter sequence 302, target sequence 304, and termination sequence 306). Thus, linear RCA product 300 may serve as a template for the expression of multiple copies of the target sequence 304 once transcribed into mRNA and, optionally, translated into a protein.

The branched RCA product 400 in (E) results from the multi-primed RCA shown in (C). Primary branches 410 are similar to linear RCA product 300, comprising a primer sequence 200 and multiple, repeating monomers of promoter sequence 302, target sequence 304, and termination sequence 306. Secondary branches 420 result from the annealing of a primer to the newly-replicated, single-stranded primary branch 410 during replication of the circular DNA template 100. While shown as a relatively simple branched structure, it should be understood that, in practice, the RCA product produced by multi-primed RCA is likely to be much more complex than shown in (E), with branching continuing until dNTPs are exhausted. Branched RCA product 400 is shown merely for purposes of illustration and contrasting with linear RCA products.

RCA products may be processed to improve transfection efficiency and/or expression of the transfected target sequence in a host organism. Typically, such processing comprises breaking the RCA product into its monomeric subunits, followed by circularization or supercoiling of the subunit. The processed RCA product may then be administered to a host organism by inoculation in a suitable transfection medium.

Transfection of the unprocessed RCA product yields transfection efficiencies comparable with those of supercoiled plasmid DNA, the form of DNA most commonly used for transfection of cells. As used herein, therefore, the term "unprocessed" means not deliberately or intentionally cleaved, circularized, and/or supercoiled.

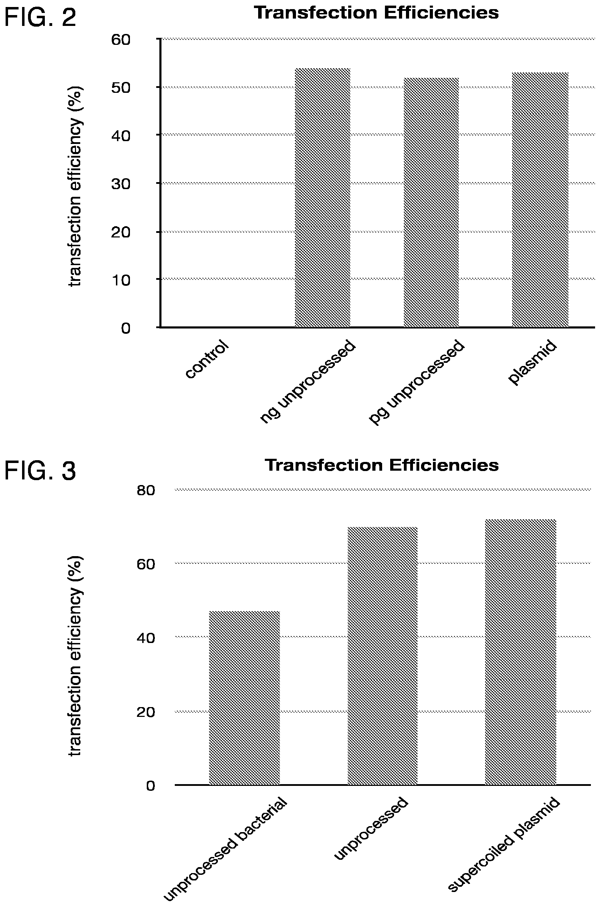

For example, FIG. 2 shows the transfection efficiencies using equal amounts of DNA that has been amplified from 1.0 nanogram and 1.0 picogram quantities of initial supercoiled plasmid DNA (pHyg-EGFP) into RCA product (10,000-fold and 10,000,000-fold amplified, respectively) and that of an equal amount of supercoiled plasmid (pHyg-EGFP). RCA was carried out for four hours at 30°C using W+W+N*N*S primers (where + denotes a locked nucleic acid (LNA) backbone and * denotes a phosphothioate backbone). 100 ng of plasmid or unprocessed RCA product were then used for reverse transfection of 30,000 HEK 293 cells using Lipofectamine™ 2000. Two days after transfection, the cells were analyzed by flow cytometry and the percentage of GFP-positive cells determined.

The efficiencies of both 10,000-fold amplified RCA product ("ng unprocessed," approximately 54%) and 10,000,000-fold amplified RCA product (("pg unprocessed," approximately 52%) are equivalent to that of supercoiled plasmid ("plasmid," approximately 53%). Consistent results were obtained in eight independent studies. "Control" results represent untransfected cells.

FIG. 3 shows the transfection efficiencies of unprocessed RCA product prepared from DNA amplified directly from pHyg-EGFP-transformed bacterial colonies containing the circular plasmid to be amplified, unprocessed RCA product prepared from a purified circular DNA template, and supercoiled plasmid DNA. The transformed bacterial colonies were resuspended in 10 microliters of Luria broth. 0.5 microliter was then added to 9.5 microliters of GenomiPhi™ sample buffer, heated to 95°C for two minutes and cooled to 4°C on ice. Nine microliters of 2x reaction buffer and 1 microliter of phi29 enzyme mix was added and incubated at 30°C for 90 minutes prior to heat inactivation at 65°C for 10 minutes. As described above, 100 ng of plasmid or unprocessed RCA DNA were then used for reverse transfection of 30,000 HEK 293 cells using Lipofectamine™ 2000 and the percentage of GFP-positive cells determined two days later by flow cytometry.

The unprocessed RCA product and supercoiled plasmid RCA product yielded similar transfection efficiencies (approximately 70% and approximately 72%, respectively). Even the unprocessed RCA product prepared from bacterial colony DNA, which allows for elimination of all plasmid DNA purification steps before transfection, yielded a transfection efficiency of approximately 48%.

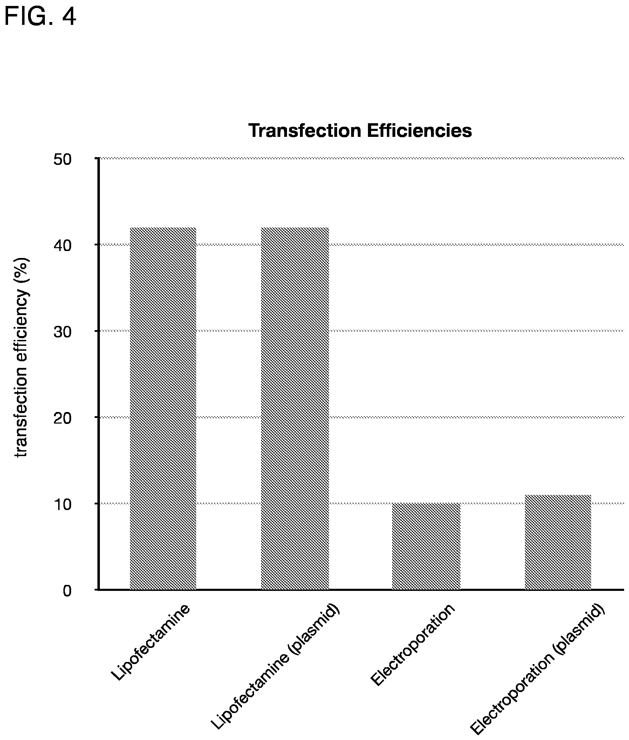

FIG. 4 shows the transfection efficiencies of unprocessed RCA product and plasmid DNA product using two common transfection techniques (lipofection using Lipofectamine™ and electroporation). Lipofection was carried out as described above. Electroporation was carried out using an Amaxa Nucleofector™ electroporator (buffer V, program Q01), electroporating 300 ng of unprocessed RCA DNA or supercoiled plasmid into 10⁶ cells. RCA DNA was electroporated in the TempliPhi™ reaction buffer and did not undergo buffer exchange to a low conductivity buffer. Two days after transfection, the percentage of GFP-positive cells was determined by flow cytometry. Both techniques yielded very similar efficiencies, with lipofection performing better for both unprocessed RCA product and plasmid DNA product.

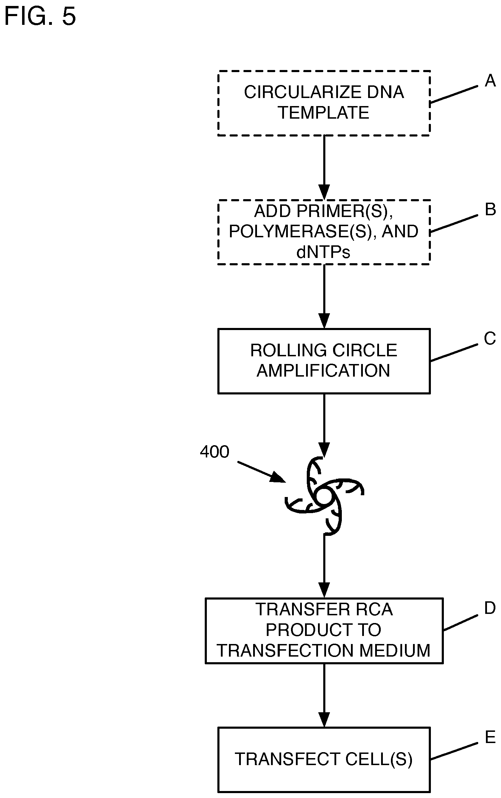

FIG. 5 shows a flow diagram of an illustrative method for preparing and using unprocessed RCA DNA product according to an embodiment of the invention. At A, the nucleic acid template (here, DNA) is circularized, if it is not so already. The DNA template may be a plasmid construct that has been created by standard molecular biology techniques, it can be created synthetically, or it can be created from purified components that are ligated or otherwise circularized together to form a circular construct. At B, one or more species of primer, one or more polymerases, and deoxynucleotide triphosphates (dNTPs) are added to the circular DNA template. In the case that the DNA template, primer(s), polymerase(s), and dNTPs are provided in a pre-combined form, such addition may be unnecessary.

In some embodiments, modified nucleotides (e.g., non-natural nucleotides or nucleotide analogs wherein one or more of the nucleotide's base, phosphate, or sugar have been modified) may be employed, some of which may render their resulting DNA or RNA resistant to nuclease activity. Such non-natural nucleotides include, for example, phosphorothioated nucleotides, LNAs, dUTP, dITP, rNTP, 5-methyl dCTP, 2-amino-dATP, 2-thio-dTTP, 4'-thio-dTTP, 4'-thio-dCTP, and deaza-dGTP. Phosphorothioated nucleotides are particularly preferred.

In known RCA methods, only the oligonucleotide primer(s) may comprise such modified nucleotides, as processing of the RCA product itself typically employs the use of nucleases to break the RCA product into monomeric subunits before their use. The use of the RCA product in its unprocessed form, however, is not subject to such limitation. Accordingly, the production of nuclease-resistant RCA product improves the product's overall stability and effectiveness, which may be particularly important where the unprocessed RCA product is to be used in a DNA vaccine.

Modified nucleotides may also be used to alter the properties of the RCA product relative to RCA product from unmodified dNTPs. Certain nucleotide analogs may give better results in vivo. For instance, modified nucleotides may make an RCA product that is transcribed well in vivo, but is not used as a template by DNA recombination systems. This could be beneficial in preventing RCA product recombination into the cellular genome, and force it to be retained as an extracellular transient element.

At C, the circular nucleic acid template is replicated by RCA to yield an unprocessed RCA product 400. At D, the unprocessed RCA product 400 is transferred directly (i.e., without processing typical of RCA products, such as deliberate or intentional cleaving, circularization, and/or supercoiling) to a suitable transfection medium. As will be understood, the transfection medium used will vary depending upon the transfection method chosen and the cells to be transfected. For example, the transfection medium may be a buffer, such as HEPES-buffered saline solution (HeBS). The transfection medium may also be a cationic polymer, such as DEAE-dextran or polyethylenimine. Alternatively, the transfection medium may be one or more of any number of commercially-available products, such as Lipofectamine™, HilyMax, FuGENE^{®}, jetPEI™, Effectene™, and DreamFect™. In the case that the unprocessed RCA product is to be used as a DNA vaccine, the medium into which the unprocessed RCA product is transferred must be physiologically-acceptable, but may also contain one or more adjuvants to augment the vaccinated organism's immune response.

Unprocessed RCA products may also be delivered to a host cell or organism using a virus. Thus, as used herein, "transfection" includes the use of a virus in delivering unprocessed RCA product.

Finally, at E, one or more cells are transfected with the unprocessed RCA product. In the case of a DNA vaccine, this may comprise the intramuscular injection of the unprocessed RCA product. Additional methods may be employed to increase uptake efficiency. Such methods include, for example, dermal abrasion, electroporation, ultrasound, and particle-mediated projectile transfection. In the case of tissue culture cell transfection, this may comprise tissue culture cells either in suspension or adhered to a matrix.

### Example 1 - Production of an Unprocessed RCA DNA Product

Supercoiled DNAplasmid pHygGFP is prepared by standard methods at a concentration of 100 ng/ microliter in TE buffer. An RCA reaction is assembled using 2.5 mL 2xR buffer (100 mM Tris:HCl, pH = 8.2; 150 mM KCl; 20 mM MgCl₂, 0.02% TWEEN^{®} 20, 2 mM DTT, 2 mM dNTP), 2.5 mL P buffer (10 mM Tris:HCl, pH = 8.2; 0.5 mM EDTA; 0.01% TWEEN^{®} 20; 0.08 mM random hexamer containing two phosphorothioate bonds at the 3' end), 0.1 mL of 1 mg/mL Phi29 DNA polymerase, and 250 ng supercoiled pHygGFP plasmid. The RCA reaction mixture is incubated at 30°C for 16 hours to allow isothermal DNA amplification to occur and then heated at 65°C for 20 minutes to inactivate the DNA polymerase.

The reaction may be assayed using PicoGreen^{®} dye binding according to the manufacturer's instructions. Typical concentrations are approximately 0.6 mg/mL, representing a greater than 10,000-fold increase (i.e., from 250 ng to 3 mg).

### Example 2 - Production of an Unprocessed RCA DNA Product

An alternative method of producing unprocessed RCA DNA product, similar to that in Example 1, may be employed. Here, the reaction mix comprises:
2.5 mL 2xR buffer;
2.5 mL thioated random hexamer in TE buffer (final concentration is 40 µM primer);
20 µL 100 mM dNTP;
5 µL 1M DTT;
50 µL 1M MgCl₂;
100 µL 1 mg/mL Phi29 DNA polymerase; and
250 ng pHygGFP supercoiled plasmid.

The reaction is incubated at 30°C for 17 hours and then heated at 65°C for 20 minutes to inactivate the enzyme. The reaction may be assayed using PicoGreen^{®} dye binding according to the manufacturer's instructions.

### Example 3 - Preparation of Supercoiled Plasmid DNA

The supercoiled plasmid DNA in either of Examples 1 or 2 may be prepared as follows using the EndoFree Plasmid Giga Kit available from Qiagen.

Transformed DH5alpha bacteria carrying the pHygGFP plasmid are grown overnight in a fermentor at 37°C in TB media. Bacterial cells are harvested by centrifugation at 6000 x g for 15 min at 4°C. The bacterial pellet is resuspended in 125 mL of Buffer P1. 125 mL of Buffer P2 is added, mixed thoroughly by vigorously inverting 4-6 times, and incubated at room temperature for 5 min. 125 mL chilled Buffer P3 is added and mixed thoroughly by vigorously inverting 4-6 times. Mixing continues until white, fluffy material has formed and the Lysate is no longer viscous. The lysate is poured into a QIAfilter Giga Cartridge and incubated at room temperature for 10 min.

The vacuum source is activated and, after all liquid has been pulled through, deactivated. The QIAfilter Cartridge is left attached. 50 mL Buffer FWB2 is added to the QIAfilter Cartridge and the precipitate gently stirred using a sterile spatula. The vacuum source is activated until the liquid has been pulled through completely.

30 mL Buffer ER is added to the filtered lysate, mixed by inverting the bottle approximately 10 times, and incubated on ice for 30 min. QIAGEN-tip 10000 is equilibrated by applying 75 mL Buffer QBT, and allowing the column to empty by gravity flow. The filtered lysate is applied onto the QIAGEN-tip and allowed to enter the resin by gravity flow. The QIAGEN-tip is washed with a total of 600 mL Buffer QC.

DNA is eluted with 100 mL Buffer QN and precipitated by adding 70 mL (0.7 volumes) room-temperature isopropanol. The solution is mixed and centrifuged immediately at ≥15,000 x g for 30 min at 4°C. The supernatant is carefully decanted and the DNA pellet washed with 10 mL of endotoxin-free room-temperature 70% ethanol (by adding 40 mL of 96-100% ethanol to the endotoxin-free water supplied with the kit) and centrifuged at ≥15,000 x g for 10 min. The supernatant is carefully decanted without disturbing the pellet and the pellet air-dried for 10-20 min. The DNA is then re-dissolved in a suitable volume of endotoxin-free Buffer TE.

The reaction product may be quantified using UV absorption and PicoGreen^{®} according to the manufacturer's instructions.

### Example 4 - Polyethylenimine Transfection of Unprocessed RCA DNA Product

Polyethylenimine (PEI) condenses plasmid DNA into positively charged particles that interact with the anionic surface of a cell. After entering the cell through endocytosis, the high charge density of the polymer causes lysosomal rupturing, releasing the DNA into the cytosol and permitting migration into the nucleus.

Unprocessed RCA product, such as that prepared in accordance with Example 1 above, may be used directly after amplification or after DNA precipitation and resuspension in an appropriate buffer.

Cells to be transfected (e.g., HEK 293) are grown following standard protocols to a concentration of up to about 3.5 × 10⁶ cells/mL, preferably between about 5 × 10⁵ cells/mL and about 1 × 10⁶ cells/mL. Cells are preferably transfected during growth phase.

Cells are seeded at a concentration between about 1 × 10⁶ cells/mL and about 2 × 10⁶ cells/mL in half the target volume. Once brought to full volume, as described below, the density will be between about 0.5 × 10⁶ cells/mL and about 1 × 10⁶ cells/mL.

A DNA complex is prepared using 2.5 micrograms of DNA per mL of culture. A 1:3 ration of DNA to PEI (w/w) is combined in 150 mM NaCl at a volume equal to approximately 5% of the culture volume. Atypical DNA complex for a 100 mL cell culture mix may comprise, for example, 0.5 mL 0.5 mg/mL DNA in TE, 1.17 mL 450 mM NaCl in TE, and 3.33 mL PEI. The complex is incubated for 10 minutes and added to the cell culture, approximately one hour after seeding. The combined culture is incubated for four hours and then brought to full volume by adding an equal volume of media.

The culture or media is harvested four to seven days post-transfection. In the case that the unprocessed RCA DNA product comprises the GFP gene, as in Example 1 above, the cells may be monitored in real time to assess GFP production. Alternatively, protein can be purified from the harvested cultures. Fluorescence activated cell sorting (FACS) analysis may also be performed.

This written description uses examples to disclose the invention and to enable any person skilled in the art to practice the invention, including making and using the disclosed compositions and performing the disclosed methods. The patentable scope of the invention is defined by the claims, and may include other examples that occur to those skilled in the art. Such other examples are intended to be within the scope of the claims if they have elements that do not differ from the literal language of the claims or if they include equivalent elements with insubstantial differences from the literal language of the claims.

## Claims

1. A method of producing a rolling circle amplification (RCA) product for transfection into a living cell, the method comprising:
preparation of a purified double stranded DNA (dsDNA) circular template;
combining at least one circular template with at least one oligonucleotide primer, at least a portion of which is complementary to a portion of the circular template;
annealing the oligonucleotide primer to the circular template;
adding to the annealed primer and circular template at least one polymerase and a quantity of deoxyribonucleotide triphosphates (dNTPs);
replicating the template by RCA, with DNA polymerase displacing newly-replicated, single stranded DNA from the circular DNA template, resulting in a linear concatemer of the sequence of the circular DNA template, wherein primers bind to the displaced single-stranded DNA and complete the double stranded RCA product; and
transferring the RCA product directly and unprocessed to a transfection medium.

2. The method of claim 1, wherein the DNA polymerase exhibits 3'-5' exonuclease activity or strand displacement activity and is preferably selected from a group consisting of: bacteriophage Phi29 DNA polymerase, Tts DNA polymerase, phage M2 DNA polymerase, Klenow fragment of DNA polymerase I, T5 DNA polymerase, PRD1 DNA polymerase, T4 DNA polymerase holoenzyme, T7 DNA polymerase, and Bst DNA polymerase.

3. The method of claim 1, wherein the DNA polymerase does not exhibit 3'-5' exonuclease activity.

4. The method of claim3, wherein the DNA polymerase is selected from a group consisting of: Taq polymerase, Tfl polymerase, Tth polymerase, eukaryotic DNA polymerase alpha, and DNA polymerases modified to eliminate 3'-5' exonuclease activity.

5. The method according to one or more of the above claims, wherein at least a portion of the dNTPs are modified nucleotides selected from a group consisting of: phosphorothioated nucleotides, locked nucleic acids (LNAs), dUTP, dITP, rNTP, 5-methyl dCTP, 2-amino-dATP, 2-thio-dTTP, 4'-thio-dTTP, 4'-thio-dCTP, and deaza-dGTP.

6. The method according to one or more of the above claims, wherein the template is supercoiled plasmid DNA and includes at least one promoter sequence, at least one target sequence, and at least one termination sequence, wherein the promoter sequence and the termination sequence preferably are eukaryotic sequences, and wherein the target sequence codes for an expression product capable of eliciting an immune response in a host organism.

7. The method of claim 6, wherein the target sequence codes for at least one of the following: a protein, such as a surface antigen, a messenger RNA (mRNA) sequence, a non-coding RNA sequence, a micro RNA (miRNA) sequence, a small interfering RNA (siRNA) sequence, and a monoclonal antibody (mAb) chain.

8. The method of claims 6 or 7, wherein the target sequence is derived from at least one of: a bacterium, a virus, a fungus, a parasitic organism, or a non-parasitic organism.

9. The method according to one or more of the above claims, wherein the template comprises at least two target sequences coding for different expression products, each expression product being capable of eliciting an immune response in a host organism.

10. A vaccine comprising at least one unprocessed rolling circle amplification (RCA) product produced according to one or more of the claims 1-9, suitable for administration to an organism.

11. The vaccine of claim 10, wherein the unprocessed RCA product comprises modified nucleotides selected from a group consisting of: phosphorothioated nucleotides, locked nucleic acids (LNAs), dUTP, dITP, rNTP, 5-methyl dCTP, 2-amino-dATP, 2-thio-dTTP, 4'-thio-dTTP, 4'-thio-dCTP, and deaza-dGTP.

12. The vaccine of claim 10, wherein the unprocessed RCA product comprises at least one target sequence coding for an expression product capable of eliciting an immune response in the organism.

## Patentansprüche

1. Verfahren für die Herstellung eines Rollkreisverstärkungs- (RCA-) Produkts zur Transfektion in eine lebende Zelle, wobei das Verfahren Folgendes umfasst:
Herstellen eines kreisförmigen Templates gereinigter doppelsträngiger DNA (dsDNA);
Kombinieren mindestens eines kreisförmigen Templates mit mindestens einem Oligoponucleotidprimer, von dem mindestens ein Teil einem Teil des kreisförmigen Templates komplementär ist;
Annealing des Oligoponucleotidprimers an das kreisförmige Template;
Zugeben zu dem Annealing durchgemachten Primer und kreisförmigen Template mindestens einer Polymerase und einer Menge an Deoxyribonucleotidtriphosphaten (dNTPs);
Replizieren des Templates durch RCA, wobei die DNA-Polymerase neu replizierte einsträngige DNA aus dem kreisförmigen DNA-Template verdrängt, was zu einem linearen Konkatemer der Sequenz des kreisförmigen DNA-Templates führt, wobei Primer sich an die verdrängte einsträngige DNA binden und das doppelsträngige RCA-Produkt vervollständigen; und
Übertragen des RCA-Produkts direkt und unverarbeitet zu einem Transfektionsmedium.

2. Verfahren nach Anspruch 1, wobei die DNA-Polymerase eine 3'-5'-Exonucleaseaktivität oder Strangverdrängungsaktivität aufweist und bevorzugt aus einer Gruppe ausgewählt wird bestehend aus: Bakteriophage Phi29-DNA-Polymerase, Tts DNA-Polymerase, Phage M2 DNA-Polymerase, Klenowfragment der DNA-Polymerase I, T5 DNA-Polymerase, PRD1 DNA-Polymerase, T4 DNA-Polymeraseholoenzym, T7 DNA-Polymerase und Bst DNA-Polymerase.

3. Verfahren nach Anspruch 1, wobei die DNA-Polymerase keine 3'-5'-Exonucleaseaktivität aufweist.

4. Verfahren nach Anspruch 3, wobei die DNA-Polymerase aus der Gruppe ausgewählt wird bestehend aus Taq-Polymerase, Tfl-Polymerase, Tth-Polymerase, eukatiotischer DNA-Polymerase-alpha und DNA-Polymerasen, die zum Eliminieren der 3'-5'-Exonucleaseaktivität modifiziert sind.

5. Verfahren nach einem oder mehreren der obigen Ansprüche, wobei mindestens ein Teil der dNTP modifizierte Nucleotide sind ausgewählt aus einer Gruppe bestehend aus: phosphorthioierten Nucleotiden, verriegelten Nucleinsäuren (LNA), dUTP, dITP, rNTP, 5-Methyl-dCTP, 2-Amino-dATP, 2-Thio-dTTP, 4'-Thio-dTTP, 4'-Thio-dCTP und Deaza-dGTP.

6. Verfahren nach einem oder mehreren der obigen Ansprüche, wobei das Template supercoiled Plasmid-DNA ist und mindestens eine Promotorsequenz, mindestens eine Targetsequenz und mindestens eine Terminationssequenz umfasst, wobei die Promotorsequenz und die Terminationssequenz bevorzugt eukaryotische Sequenzen sind und wobei die Targetsequenz für ein Expressionsprodukt codiert, das in der Lage ist, eine Immunantwort in einem Wirtsorganismus hervorzurufen.

7. Verfahren nach Anspruch 6, wobei die Targetsequenz für mindestens eines der Folgenden codiert: ein Protein wie beispielsweise ein Oberflächenantigen, eine Messenger-RNA (mRNA)-Sequenz, eine nicht codierende RNA-Sequenz, eine Mikro-RNA-(miRNA-) Sequenz, eine kleine Interferenz-RNA-(siRNA-) Sequenz und eine monoklonale Antikörper- (mAb-) Kette.

8. Verfahren nach Anspruch 6 oder 7, wobei die Targetsequenz von mindestens einem abgeleitet ist von: einem Bakterium, einem Virus, einem Pilz, einem parasitischen Organismus oder einem nichtparasitischen Organismus.

9. Verfahren nach einem oder mehreren der obigen Ansprüche, wobei das Template mindestens zwei Targetsequenzen umfasst, die für verschiedene Expressionsprodukte codieren, wobei jedes Expressionsprodukt in der Lage ist, eine Immunantwort in einem Wirtsorganismus hervorzurufen.

10. Impfstoff umfassend mindestens ein unverarbeitetes Rollkreisverstärkung- (RCA-) Produkt, das nach einem oder mehreren der Ansprüche 1 - 9 hergestellt und für die Verabreichung einem Organismus geeignet ist.

11. Impfstoff nach Anspruch 10, wobei das unverarbeitete RCA-Produkt modifizierte Nucleotide umfasst ausgewählt aus einer Gruppe bestehend aus: phosphorthioierten Nucleotiden, verriegelten Nucleinsäuren (LNA), dUTP, dITP, rNTP, 5-Methyl-dCTP, 2-Amino-dATP, 2-Thio-dTTP, 4'-Thio-dTTP, 4'-Thio-dCTP und Deaza-dGTP.

12. Impfstoff nach Anspruch 10, wobei das unverarbeitete RCA-Produkt mindestens eine Targetsequenz umfasst, die für ein Expressionsprodukt codiert, das in der Lage ist, eine Immunantwort in einem Organismus hervorzurufen.

## Revendications

1. Procédé de production d'un produit d'amplification par cercle roulant (RCA) pour transfection dans une cellule vivante, le procédé comprenant :
la préparation d'une matrice circulaire d'ADN double brin (ADNds) purifiée ;
la combinaison d'au moins une matrice circulaire avec au moins une amorce d'oligonucléotide, dont au moins une partie est complémentaire d'une partie de la matrice circulaire ;
l'annelage de l'amorce d'oligonucléotide avec la matrice circulaire ;
l'addition à l'amorce et à la matrice circulaire annelées d'au moins une polymérase et d'une certaine quantité de triphosphates de désoxyribonucléotides (dNTP) ;
la réplication de la matrice par RCA avec de l'ADN polymérase en déplaçant de l'ADN monobrin récemment répliqué de la matrice d'ADN circulaire, ce qui entraîne un concatémère linéaire de la séquence de la matrice d'ADN circulaire, dans lequel les amorces se lient à l'ADN monobrin déplacé et complètent le produit RCA double brin ; et
le transfert du produit RCA directement et sans traitement à un support de transfection.

2. Procédé selon la revendication 1, dans lequel l'ADN polymérase présente une activité de 3'-5' exonucléase ou une activité de déplacement de brin et est de préférence sélectionnée dans un groupe constitué d'une ADN polymérase de bactériophage Phi29, d'une ADN polymérase de Tts, d'une ADN polymérase de phage M2, d'un fragment d'ADN polymérase I de Klenow, d'une ADN polymérase de T5, d'une ADN polymérase de PRD1, d'un holoenzyme d'ADN polymérase de T4, d'une ADN polymérase de T7 et d'une ADN polymérase de Bst.

3. Procédé selon la revendication 1, dans lequel l'ADN polymérase ne présente pas d'activité de 3'-5' exonucléase.

4. Procédé selon la revendication 3, dans lequel l'ADN polymérase est sélectionnée dans un groupe constitué d'une Taq polymérase, d'une Tfl polymérase, d'une Tth polymérase, d'une ADN polymérase eucaryotique alpha et d'ADN polymérase modifiée pour éliminer l'activité de 3'-5' exonucléase.

5. Procédé selon une ou plusieurs des revendications précitées, dans lequel au moins une partie des dNTP sont des nucléotides modifiés sélectionnées dans un groupe constitué de nucléotides phosphorothioatés, d'acides nucléiques bloqués (LNA), de dUTP, de dITP, de rNTP, de 5-méthyl dCTP, de 2-amino-dATP, de 2-thio-dTTP, de 4'-thio-dTTP, de 4'thio-dCTP et de déaza-dGTP.

6. Procédé selon une ou plusieurs des revendications précitées, dans lequel la matrice est de l'ADN de plasmide surenroulé et comprend au moins une séquence promoteur, au moins une séquence cible et au moins une séquence terminale, dans lequel la séquence promoteur et la séquence terminale sont de préférence des séquences eucaryotiques et dans lequel la séquence cible code pour un produit d'expression capable de provoquer une réponse immunitaire dans un organisme hôte.

7. Procédé selon la revendication 6, dans lequel la séquence cible code pour au moins l'une des suivantes : une protéine, telle qu'un antigène de surface, une séquence d'ARN messager (ARNm), une séquence d'ARN non codante, une microséquence d'ARN (ARNmi), une petite séquence d'ARN interférente (ARNsi) et une chaîne d'anticorps monoclonal (Abm).

8. Procédé selon les revendication 6 ou 7, dans lequel la séquence cible est tirée d'au moins l'un des organismes suivants : une bactérie, un virus, un champignon, un organisme parasite ou un organisme non parasite.

9. Procédé selon une ou plusieurs des revendications précitées, dans lequel la matrice comprend au moins deux séquences cibles codant pour différents produits d'expression, chaque produit d'expression étant capable de provoquer une réponse immunitaire dans un organisme hôte.

10. Vaccin comprenant au moins un produit d'amplification par cercle roulant (RCA) non traité produit selon une ou plusieurs des revendications 1 à 9, convenant à une administration à un organisme.

11. Vaccin selon la revendication 10, dans lequel le produit RCA non traité comprend des nucléotides modifiés sélectionnés dans un groupe constitué de nucléotides phosphorothioatés, d'acides nucléiques bloqués (LNA), de dUTP, de dITP, de rNTP, de 5-méthyl dCTP, de 2-amino-dATP, de 2-thio-dTTP, de 4'-thio-dTTP, de 4'thio-dCTP et de déaza-dGTP.

12. Vaccin selon la revendication 10, dans lequel le produit RCA non traité comprend au moins une séquence cible codant pour un produit d'expression capable de provoquer une réponse immunitaire dans l'organisme.
